# FASCICULE DE BREVET EUROPEEN

(11) **EP 1 255 543 B1**
(45) Date de publication et mention de la délivrance du brevet: **01.09.2010**
(21) Numéro de dépôt: 01903998.1
(22) Date de dépôt: 30.01.2001
(51) Int. Cl.: A61K 31/192, A61K 31/166, A61K 31/196, A61K 31/05, A61K 31/28, A61K 31/381, A61K 31/382, A61K 31/343, A61P 17/00, A61P 17/10, A61P 31/04

(54) **UTILISATION DE COMPOSES DE TYPE RETINOIDES EN TANT QU'AGENTS ANTI-BACTERIENS CONTRE STAPHYLOCOCCUS AUREUS**
VERWENDUNG VON RETINOID-TYP VERBINDUNGEN ALS ANTIBAKTERIELLE MITTEL GEGEN STAPHYLOCOCCUS AUREUS
USE OF RETINOID-TYPE COMPOUNDS AS ANTIBACTERIAL AGENTS AGAINST STAPHYLOCOCCUS AUREUS

(30) Priorité: 31.01.2000 FR 0001206
(43) Date de publication de la demande: 13.11.2002
(73) Titulaire: GALDERMA RESEARCH & DEVELOPMENT, 06410 Biot (FR)
(72) Inventeur: VOEGEL, Johannes, F-06740 Châteauneuf/Grasse (FR); CAVEY, Marie-Thérèse, F-06530 Peymeinade (FR)
(74) Mandataire: Allab, Myriam
(86) Numéro de dépôt international: PCT/FR2001/000280
(87) Numéro de publication internationale: WO 2001/056554

(56) Documents cités:
- EP-A- 0 199 636
- EP-A- 0 292 348
- EP-A- 0 658 553
- EP-A- 0 679 631
- EP-A- 0 740 937
- EP-A- 0 879 814
- WO-A-93/15740
- WO-A-97/09987
- WO-A-98/18440
- FR-A- 2 736 548
- FR-A- 2 761 600
- FR-A- 2 767 525
- FR-A- 2 787 322
- US-A- 6 017 938
- GOLLNICK H ET AL: "TOPICAL DRUG TREATMENT IN ACNE" DERMATOLOGY,XX,XX, vol. 196, no. 1, 1998, pages 119-125, XP000869711 ISSN: 1018-8665
- GOLLNICK ET AL.: "Topical therapy in acne" J. EUROP. ACAD. DERMATO. VENEREOL., vol. 11, no. suppl.1, 1998, pages s8-s12, XP000974550
- BERGFELD: "Topical retinoids in the treatment of acne vulgaris" J. DRUG DEV. CLIN. PRACTICE, vol. 8, no. 3, 1996, pages 151-160, XP000974558

## Description

L'invention concerne l'utilisation d'au moins un composé choisi parmi des molécules de type rétinoides pour la préparation d'une composition destinée à traiter de manière préventive ou curative les colonisations bactériennes de staphylococcus aureus, les aggravations des pathologies provoquées par ces colonisations ainsi que les surinfections cutanées induites par ces bactéries.

Les composés de type rétinoides sont des composés présentant un profil d'activité biologique analogue à celui de l'acide rétinoïque *tout-trans* ou de acide 9-cis-rétinoïque. Ces composés peuvent moduler l'expression des gènes par le biais des récepteurs de la famille de l'acide rétinoique, tels que les RARs et RXRs. Ainsi, les rétinoïdes peuvent présenter une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités de ces composés respectivement dans les domaines de la différenciation et de la prolifération cellulaire.

Ces rétinoïdes ont été déjà décrits dans de nombreuses applications pharmaceutiques, plus particulièrement dermatologiques ou cosmétiques. Dans le domaine pharmaceutique, ils ont été notamment proposés pour traiter des affections dermatologiques, rhumatismales, respiratoires, cardiovasculaires et ophtalmologiques. Dans le domaine dermatologique ou cosmétique, il a été notamment décrit dans EP 0 379 367 une méthode de traitement préventif ou curatif de la peau âgée en utilisant des rétinoïdes.

Par ailleurs, il est bien connu que la peau est couverte d'une flore responsable de toute une série de désagréments allant de la simple production d'odeur à des pathologies plus ou moins sévères comme par exemple l'acné et/ou les pellicules.

Les micro-organismes commensaux vivant sur ou dans la peau peuvent faire partie d'une microflore soit résidante (normale) soit transitoire. Les organismes résidants se développent normalement sur ou dans la peau. Leur présence est établie en profils de distribution bien définis. Les micro-organismes présents temporairement sont appelés transitoires. Habituellement, ces organismes ne se fixent pas fermement ; ils sont incapables de se multiplier et meurent normalement après quelques heures.

L'anatomie et la physiologie de la peau varient d'une partie à l'autre du corps et la microflore résidante reflète ces variations.

La plupart des bactéries de la peau sont présentes sur l'épiderme squameux superficiel, colonisant les cellules mortes ou étroitement associées aux glandes sébacées et sudoripares. Les excrétions de ces glandes fournissent de l'eau, des acides aminés, de l'urée, des électrolytes et des acides gras spécifiques servant d'éléments nutritifs principalement pour *Staphylococcus epidermidis* et des corynébactéries aérobies.

Les bactéries Gram-négatives sont généralement présentes dans les régions plus humides. Certains agents pathogènes présents sur ou dans la peau sont des résidants/transitoires colonisant les zones autour des orifices. *Staphylococcus aureus* est le meilleur exemple. Il est résidant dans les nannes et la région périanale mais est transitoire sur les autres parties du corps où il survit mal.

Théoriquement, l'épiderme n'est pas un environnement favorable pour la colonisation par les micro-organismes. Plusieurs facteurs comme le dessèchement périodique de la peau, le pH légèrement acide de la peau, la concentration élevée en chlorure sodique de la sueur, certaines substances inhibitrices naturelles (bactéricides et/ou bactériostatiques) sont responsables de ce micro-environnement hostile.

Le pH acide (4-6) de la peau, dû aux acides organiques produits par les staphylocoques et aux sécrétions des glandes sébacées et sudoripares, décourage la colonisation par de nombreux micro-organismes.

La sueur contient du chlorure sodique en une concentration qui établit des conditions hyperosmotiques à la surface de la peau et pèse osmotiquement sur la plupart des micro-organismes.

Finalement, certaines substances inhibitrices naturelles aident à contrôler la colonisation, la croissance excessive et l'infection de la surface de la peau par les micro-organismes résidants. Par exemple, les glandes sudoripares excrètent du lysozyme qui lyse *Staphylococcus epidermidis* et d'autres bactéries Gram-positives.

Les souches de *Staphylococcus aureus* sont connues comme produisant des toxines et des super antigènes, ce qui favorise l'apparition de réactions d'irritation et de processus inflammatoires. Ainsi, plus de 90% des cas de dermatite atopique présentent cette bactérie ( British Journal of Dermatology , 1998 :139 :13-16).

Par ailleurs, il a été mis en évidence que les patients atteints de dermatite atopique pouvaient agir en tant que réservoirs pour la transmission de *Staphylococcus aureus* à d'autres personnes. Les études présentées dans Pediatric Dermatology, vol 15 N°3, 194-198, 1998 suggèrent que les narines et les mains soient respectivement d'importants réservoirs et vecteurs de transmission de cette bactérie vers des peaux lésionnelles et des personnes en contacts rapprochés avec ces patients.

Il est donc important de traiter ces personnes atteintes de dermatite atopique, afin de prévenir l'aggravation de la pathologie dont ils sont atteints, et en particulier de prévenir le risque de surinfection massive par le *Staphylococcus aureus*, comme c'est le cas pour le « syndrome de la peau brûlée », mais également de prendre des mesures préventives vis à vis d'individus mis en contact avec ces patients et susceptibles de développer des infections bactériennes en particulier dans le milieu hospitalier (International Journal of Dermatology, November 1986, vol 25, N°9).

Un article publié aux pages 520 à 532 de la revue intitulée « The New England Journal of Medecine », vol 339. N°8 présente l'état actuel des connaissances sur la bactérie *Staphylococcus aureus*, son épidémiologie et les traitements des maladies dans lesquelles cette bactérie est impliquée. Il ressort de ce document que les taux de colonisation seraient plus élevés parmi les patients atteints de diabète de type 1, les utilisateurs de drogue par voie intraveineuse, les patients sous hémodialyse, les patients ayant subi une intervention chirurgicale et les patients porteurs du syndrome d'immunodéficience humaine. Les personnes présentant des défauts d'un point de vue qualitatif ou quantitatif en leukocytes feraient également partie d'une population à risque vis à vis d'une colonisation par la bactérie *Staphylococcus aureus*

Bien évidemment, si nécessaire, on sait utiliser depuis longtemps des composés comme les antibiotiques.

Mais, là encore on sait que l'utilisation de tels composés présente des aspects négatifs non négligeables. L'utilisation abusive d'antibiotiques peut aboutir à l'émergence de micro-organismes résistants sur lesquels ils n'ont plus d'efficacité. Ainsi, là encore on a besoin de composés et/ou de compositions efficaces.

Dans cette optique, il a été décrit dans FR 2736548 l'utilisation d'un rétinoïde pour la préparation d'un médicament bactérien. Cependant, les rétinoïdes utilisés sont des aldéhydes qui présentent une faible activité anti-bactérienne et qui sont, de part leur fonction aldéhyde, cytotoxiques et potentiellement allergisants.

De manière tout à fait surprenante et inattendue, la Demanderesse a trouvé que certains composés de type rétinoides ont une activité anti-bactérienne marquée, et en particulier une activité anti-bactérienne marquée vis à vis de la bactérie *Staphylococcus aureus.*

Cette découverte est à la base de la présente invention.

La présente invention a donc trait à l'utilisation d'au moins un composé de type rétinoïde dans une quantité efficace pour la préparation d'une composition destinée à traiter de manière préventive ou durative les colonisations bactériennes de staphylococcus aureus, ledit composé étant choisi parmi les composes 1 à 45 indiqués dans le tableau 1 ci-dessous

| | Tableau 1 |
|---|---|
| N° | Nom chimique |
| 1 | acide 6-[3-(1-adamantyl)-4-methoxy-5-hydroxyphenyl]-2- naphtoique |
| 2 | acide 4-[4-(6-Methoxymethoxy-4'-methyl-biphenyl-2-yl)-but-3-en-1-ynyl]-benzoïque |
| 3 | acide 4-[4-(6-Methoxy-4'-methyl-biphenyl-2-yl)-but-3-en-1-ynyl]- benzoïque |
| 4 | acide 6-[2-methyl-4-hydroxy-5-(1-adamantyl)phenyl]-2- naphtoïque |
| 5 | acide 4-[3-(3,5-Di-tert-butyl-4-oxo-cyclohexa-2,5-dienylidene)-prop-1-ynyl]-benzoïque |
| 6 | acide 2-Hydroxy-4-[4-(4'-methyl-biphenyl-2-yl)-but-3-en-1-ynyl]- benzoïque |
| 7 | acide 2-[3-(1-adamantyl)-4-meihoxyphenyl)ethynyl]-4-thiophene carboxylique |
| 8 | acide 4-[4-(4,4'-Dimethyl-biphenyl-2-yl)-but-3-en-1-ynyl]- benzoïque |
| 9 | acide 4-Hydroxy-2'-(5,5,8,8-tetramethyl-5,6,7,8-tetrahydro- naphtalen -2-yl)-[1,1';4',1"]terphenyl-4"- carboxylique |
| 10 | acide 2"-Hydroxy-2'-(5,5,8,8-tetramethyl-5,6,7,8-tetrahydro- naphtalen -2-yl)-[1,1';4',1"] terphenyl-4"-carboxylique |
| 11 | acide 6-(3-Adamantan-1-yl-5-bromo-4-hydroxy-phenyl)- naphtalene -2-carboxylique |
| 12 | acide 4-[4-(6,4'-Dimethyl-biphenyl-2-yl)-but-3-en-1-ynyl]- benzoïque |
| 13 | acide 2'-(4,4-Dimethyl-thiochroman-6-yl)-[1,1';4',1"]terphenyl-4"-carboxylique |
| 14 | acide 2'-(3-Hydroxy-5,5,8.8-tetramethyl-5,6,7,8-tetrahydro- naphtalen -2-yl)-[1,1';4',1"]terphenyl-4"- carboxylique |
| 15 | acide 4-(5,5,8,8-Tetramethyl-5,6,7,8-tetrahydro- naphto [2,3-b]thiophen-2-yl)-benzoïque |
| 16 | acide 4-[(6-hydroxy-7-(1-adamantyl)-2-naphtyl]benzoïque |
| 17 | acide 4-[2-Nonyloxyimio-2-(5,5,8,8-tetramethyl-5.6,7,8-tetrahydro-naphtalen-2-yl)-acetylamino]-benzoïque |
| 18 | acide 4-[3-Adamantan-l-yl-4-(2-methoxy-ethoxymethoxy)-phenylethynyl]-2-hydroxy- benzoïque |
| 19 | acide 4-[[3-(1-adamantyl)-4-[(2-methyl-3-hydroxypropyloxy)phenyl)ethynyl]] benzoïque |
| 20 | 2-Hydroxymethyl-5-(5,5.8.8-tetramethyl-5.6.7,8-tetrahydro-anthracen-2-yl)-phenol |
| 21 | acide 4-[4-(4'-Propyl-biphenyl-2-yl)-but-3-en-1-ynyl]- benzoïque |
| 22 | acide 2-Hydroxy-4-(3,5,5,8,8-pentamethyl-5,6,7,8-tetrahydro- naphtalen -2- |
| | ylselanylethynyl)- benzoïque |
| 23 | acide 2'-(3-Methoxy-5,5,8,8-tetramethyl-5,6,7,8-tetrahydro- naphtalen -2-yl)-[1,1';4',1"]terphenyl-4"- carboxylique |
| 24 | acide 6-[Butoxy-(5,5,8.8-tetramethyt-5,6,7,8-tetrahydro-naphtalen-2-yl)-methyl]-naphtalene-2-carboxylique |
| 25 | acide 4-[3-(1,1-Dimethyl-decyl)-4-methoxy-benzoylamino]- benzoïque |
| 26 | acide 4-(3-Adamantan-1-yl-4-hexyloxy-benzoylamino)- benzoïque |
| 27 | 6-[3-(1-adamantyl)-4-hydroxyphenyl]-2- naphtylmethanol |
| 28 | acide 2-Hydroxy-4-(5,5,8,8-tetramethyl-5,6.7.8-tetrahydro-anthracen-2-yl)-benzoïque |
| 29 | acide 2-(3-Adamantan-1-yl-4-hydroxy-phenyl)-benzofuran-5-carboxylique |
| 30 | acide 4-[6-methoxyethoxymethoxy-7-(1-adamantyl)-2-naphthyl]salicylique |
| 31 | acide (E)-4-[4-(5-Methoxymethoxy-4'-methyl-biphenyl-2-yl)-but-3-en-1-ynyl]-benzoïque |
| 32 | acide 4-[4-(3-Methoxy-4'-methyl-biphenyl-2-yl)-but-3-en-1-ynyl]-benzoïque |
| 33 | acide 4-(3,5,5.8,8-Pentamethyl-5,6.7,8-tetrahydro-naphthalen-2-ylselanylethynyl)-benzoïque |
| 34 | acide 2-Methoxymethoxy-2'-(5,5,8,8-tetramethyl-5,6,7,8-tetrahydro- naphtalen -2-yl)-[1,1';4',1"]terphenyl-4"-carboxylique |
| 35 | acide 2-Hydroxy-2'-(5,5,8,8-tetramethyl-5,6,7,8-tetrahydro- naphtalen -2-yl)-[1,1';4',1"]terphenyl-4"- carboxylique |
| 36 | acide 4-[(Z)-2-(5,6,7,8-tetrahydro-5,5,8,8-tetramethyl-2-naphtyl)-2-undecenamido]benzoïque |
| 37 | acide 4-[4-(4'-Methyl-biphenyl-2-yl)-but-3-en-1-ynyl]- benzoïque |
| 38 | acide 2'-Propoxy-5'-(5.5.8.8-tetramethyl-5,6,7,8-tetrahydro- naphtalen -2-yl)-biphenyl-4-carboxylique |
| 39 | acide 4-[2-Heptyioxyimino-2-(5.5.8.8-tetramethyl-5,6,7,8-tetrahydro- naphtalen -2-yl)- acetylamino]- benzoïque |
| 40 | amide de l'acide 6-(5,5,8,8-Tetramethyl-5,6,7,8-tetrahydro-naphthalen-2-yloxy)-naphthalene-2-carboxylique |
| 41 | (2-dimethylammo-ethyl)-amide de l'acide 6-(3-Adamantan-1-yl-4-methoxy-phenyl)-naphthalene-2-carboxylique |
| 42 | acide 2-Hydroxy-4-(5,5,8,8-tetramethyl-5,6,7,8-tetrahydro-naphtalen-2-ylselanylethynyl)-benzoïque |
| 43 | acide 2'-(3-Methoxymethoxy-5,5,8,8-tetramethyl-5,6,7,8-tetrahydro- naphtalen -2-yl)-[1,1';4',1"] terphenyl-4"-carboxylique |
| 44 | acide 3-Hydroxy-2'-(5,5,8,8-tetramethyl-5,6,7,8-tetrahydro- naphtalen -2-yl)-[1,1';4',1"] terphenyl-4"-carboxylique |
| 45 | acide 3-(5'-Adamantan-1-yl-4'-methoxy-2'-methyl-biphenyl-3-yl)-acrylique |

La présente invention a également trait à l'utilisation dans une quantité efficace d'au moins un composé choisi parmi les composés 1 à 45 du tableau 1 pour la préparation d'une composition destiné à traiter de manière préventive ou curative les aggravations des pathologies provoquées par une colonisation bactérienne de shaphylococcus aureus.

Parmi les pathologies qui peuvent être aggravées par une colonisation par la bactérie *Staphylococcus aureus* on peut citer l'impetigo ou la dermatite atopique.

Certains composés indiqués dans le tableau 1 et présentant donc une activité antibactérienne marquée présentent en outre une activité biologique de type antagoniste des récepteurs RAR C'est le cas en particulier des composés indiqués dans la liste ci-dessous :
acide 4-[4-(6-Methoxymethoxy-4'-methyl-biphenyl-2-yl)-but-3-en-1-ynyl]-benzoïque
acide 4-[4-(6-Methoxy-4'-methyl-biphenyl-2-yl)-but-3-en-1-ynyl]-benzoïque
acide 4-[6-methoxyethoxymethoxy-7-(1-adamantyl)-2-naphthyl]salicylique
acide (E)-4-[4-(5-Methoxymethoxy-4'-methyl-biphenyl-2-yl)-but-3-en-1-ynyl]-benzoïque
acide 4-[4-(3-Methoxy-4'-methyl-biphenyl-2-yl)-but-3-en-1-ynyl]-benzoïque
acide 2-Methoxymethoxy-2'-(5,5,8,8-tetramethyl-5,6,7,8-tetrahydro- naphtalen -2-yl)-[1,1';4',1"]terphenyl-4"-carboxylique
acide 4-[4-(4'-Methyl-biphenyl-2-yl)-but-3-en-l-ynyl]-benzoïque
acide 4-[4-(4'-Propyl-biphenyl-2-yl)-but-3-en-l-ynyl]-benzoïque.

Or il est connu que les composés antagonistes des récepteurs RAR induisent des effets anti-inflammatoires et immunomodulateurs. Ainsi, dans un modèle animal d'eczema chronique, des composés antagonistes des récepteurs RAR ont montré des effets d'inhibition des signes cliniques et histologiques de l'inflammation analogues à ceux induits par des glucocorticoïdes ou des immunomodulateurs de la classe des macrolides, comme le tacrolimus, sans toutefois provoquer l'apparition d'un effet secondaire tel que l'atrophie cutanée comme cela est le cas après un traitement par les glucocorticoïdes (A. Jomard et al., J. Invest. Dermatol Vol. 110, No 4, April 1998, page 577)*.*

Ainsi, les composés qui presentent une activité antibactérienne marquée vis à vis de *Staphylococcus aureus* et une activité antagoniste RAR. comme c'est le cas pour les composés précédemment cités sont particulièrement adaptés pour le traitement de la dermatite atopique car ils agissent simultanément sur deux cibles qui sont les anomalies du système immunitaire et la prolifération de la bactérie *Staphylococcus aureus.*

La présente invention a également trait à une utilisation dans une quantité efficace d'au moins un composé choisi parmi les composés 1 à 45 du tableau 1 pour la préparation d'une composition destinée à traiter de manière préventive ou curative les surinfections cutanées induites par une colonisation bactérienne de shaphylococcus aureus.

Parmi les surinfections cutanées induites par une colonisation bactérienne par la bactérie *Staphylococcus aureus* on peut citer le « syndrome de la peau brûlée ».

L'activité anti-bacténenne marquée vis à vis de la bactérie *Staphylococcus aureus* des composés de la présente invention peut être mise en évidence par des tests comme ceux décrits dans la publication intitulée « National committee for clinical laboratory standards » Vol. 13, N°25, décembre 1993, Methods for dilution antimicrobial susceptibility tests for bacteria that grow aerobically-third édition, approved standard ».

La bactérie *Staphylococcus aureus* CIP52.1 peut être utilisée afin de déterminer pour chaque composé testé la concentration minimale inhibitrice pour la bactérie ou CMI exprimée en µM.

Dans la présente invention on entend par composé présentant une activité anti-bactérienne marquée vis à vis de la bactérie *Staphylococcus aureus* tout composé qui présente dans le test d'activité anti-bactérienne indiqué ci-dessus une concentration minimale d'inhibition de la bactérie inférieure ou égale à 5 µM. De préférence, le composé présentant une activité anti-bactérienne marquée vis à vis de la bactérie *Staphylococcus aureus* présente une concentration minimale inhibitrice de la bactérie inférieure ou égale à 2,5 µM et avantageusement inférieure ou égale à 2 µM.

Les résultats de tests des composés selon l'invention sont présentés dans l'exemple 1 de la présente demande.

L'administration des composés selon l'invention peut être effectuée par voie entérale, parentérale, topique ou oculaire.

Par voie entérale, les compositions pharmaceutiques peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Les composés selon l'invention sont généralement administrés à une dose journalière d'environ 0.001mg/kg à 500mg/kg et de préférence d'environ 0.01mg/kg à 50mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter sous forme anhydre, sous forme aqueuse, ou bien sous forme d'émulsions.

Par voie oculaire, ce sont principalement des collyres.

Les composés de l'invention sont utilisés dans ces compositions pour la voie topique ou oculaire à une concentration généralement comprise entre 0,001 % et 10 % en poids, de préférence entre 0,01 et 1 % en poids, par rapport au poids total de la composition.

De préférence, l'administration du composé ou de la composition selon l'invention est effectuée par voie topique.

Les composés selon l'invention trouvent également une application dans le domaine cosmétique, en particulier dans l'hygiène corporelle et capillaire et plus particulièrement pour nettoyer la peau ou corriger son odeur.

D'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

Les compositions selon l'invention peuvent bien entendu en outre contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféique ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11-trynoïque, leurs esters et amides ou des rétinoïdes, les vitamines D ou leurs dérivés, des corticostéroïdes, des anti-radicaux libres, des α-hydroxy ou α-céto acides ou leurs dérivés ou bien encore des bloqueurs de canaux ioniques.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B. des anti-oxydants, tels que l'α-tocophérol, le butylhydroxyanisole ou le butylhydroxytoluène

Bien entendu, l'homme du métier veillera a choisir le ou les éventuels composés à ajouter à ces compositions de telle manière que les propriétés avantageuses attachées intrinsèquement à la présente invention ne soient pas, ou substantiellement pas, altérées par l'addition envisagée.

On va maintenant donner. à titre d'illustration et sans aucun caractère limitatif, plusieurs exemples de résultats de tests d'activité anti-bactérienne vis à vis de la bactérie *Staphylococcus aureus* des composés selon l'invention et d'exemples comparatifs, ainsi que diverses formulations concrètes à base de tels composés. Dans ce qui suit ou ce qui précède, les pourcentages sont donnés en poids sauf mention contraire.

### EXEMPLE 1

Dans cet exemple, on a illustré divers résultats de tests biologiques qui montrent les propriétés anti-bactériennes vis à vis de la bactérie *Staphylococcus aureus* des composés de l'invention.

La méthode utilisée pour déterminer la concentration minimale inhibitrice ou CMI est celle décrite dans la publication intitulée « National committee for clinical laboratory standards » Vol. 13. N°25, décembre 1993 ; Methods for dilution antimicrobial susceptibility tests for bacteria that grow aerobically-third edition, approved standard ». Cette concentration minimale inhibitrice donne une indication sur l'effet bactériostatique des composés testés, c'est à dire la capacité de ces composés à arrêter la prolifération de la bactérie.

Chaque composé de l'invention a été testé au moins à trois reprises. La moyenne des résultats de test a été calculée ainsi que l'écart-type. Le composé de référence utilisé est la Gentamycine. Les résultats obtenus sont regroupés dans le tableau suivant :

| **Tableau II** | **Résultats CMI (**µ**M)** | |
|---|---|---|
| **Composé** | **Moyenne** | **Ecart-type** |
| **Gentamycine** | 0.11 | 0.06 |
| **1** | 1.04 | 0.36 |
| **2** | 1.04 | 0.36 |
| **3** | 1.04 | 0.36 |
| **4** | 1.25 | 0.00 |
| **5** | 1.25 | 0.00 |
| **6** | 1.25 | 0.00 |
| **7** | 1.66 | 0.72 |
| **8** | 1.66 | 0.72 |
| **9** | 1.66 | 0.72 |
| **10** | 1.66 | 0.72 |
| **11** | 2.08 | 0.72 |
| **12** | 2.08 | 0.72 |
| **13** | 2.08 | 0.72 |
| **14** | 2.08 | 0.72 |
| **15** | 2.50 | 0.00 |
| **16** | 2.50 | 0.00 |
| **17** | 2.50 | 0.00 |
| **18** | 2.50 | 0.00 |
| **19** | 2.50 | 0.00 |
| **20** | 2.50 | 0.00 |
| **21** | 2.50 | 0.00 |
| **22** | 2.50 | 0.00 |
| **23** | 2.50 | 0.00 |
| **24** | 3.33 | 1.44 |
| **25** | 3.33 | 1.44 |
| **26** | 3.33 | 1.44 |
| **27** | 3.33 | 1.44 |
| **28** | 3.33 | 1.44 |
| **29** | 3.33 | 1.44 |
| **30** | 3.33 | 1.44 |
| **31** | 3.33 | 1.44 |
| **32** | 3.33 | 1.44 |
| **33** | 3.33 | 1.44 |
| **34** | 3.33 | 1.44 |
| **35** | 3.33 | 1.44 |
| **36** | 4.16 | 1.44 |
| **37** | 4.16 | 1.44 |
| **38** | 4.16 | 1.44 |
| **39** | 5.00 | 0.00 |
| **40** | 5.00 | 0.00 |
| **41** | 5.00 | 4.33 |
| **42** | 5.00 | 0.00 |
| **43** | 5.00 | 4.33 |
| **44** | 5.00 | 0.00 |
| **45** | 5.00 | 0.00 |

Ces résultats montrent l'activité anti-bactérienne vis à vis de la bactérie *Staphylococcus aureus* des composés selon l'invention.

### EXEMPLE 2

Dans cet exemple, on a illustré divers résultats de tests biologiques qui montrent la supériorité des propriétés anti-bactériennes vis à vis de la bactérie *Staphylococcus aureus* des composés de l'invention par rapport à des composés rétinaldéhydes qui sont décrits dans la demande de brevet français de Pierre Fabre N° FR 2 736 548.

La méthode utilisée pour déterminer les CMI est la même que celle décrite dans l'exemple 1 de la présente demande de brevet.

La méthode utilisée pour déterminer la concentration minimale bactéricide ou CMB correspond à celle décrite dans Bactéricidie, P. Courvalin et al., Editions Maloine, 1990, chapitre 7 page 335.

Cette concentration minimale bactéricide donne une indication sur l'effet bactéricide des composés testés, c'est à dire la capacité de ces composés à tuer la bactérie.

Chaque composé de l'invention et chaque exemple comparatif issu de la demande de brevet de Pierre Fabre N° FR 2 736 548 a été testé au moins à trois reprises. La moyenne des résultats de test a été calculée ainsi que l'écart-type. Le composé de référence utilisé est la Gentamycine. Les résultats obtenus sont regroupés dans les tableaux suivants :
L'exemple comparatif 1 est le rétinaldéhyde tout trans.
L'exemple comparatif 2 est le 9-cis retinaldéhyde.
L'exemple comparatif 3 est le 13-cis retinaldéhyde.

### Résultats CMI (µM) :

| **Tableau III** | **Résultats CMI (**µ**M)** | |
|---|---|---|
| **Composé** | **Moyenne** | **Ecart-type** |
| Gentamycme | 0.14 | 0.06 |
| Composé 1 | 1.30 | 0.45 |
| Composé 5 | 3.30 | 2.57 |
| Composé 2 | 2 45 | 1.37 |
| Exemple comparatif 1 | 11.64 | 5.85 |
| Exemple comparatif 2 | 11.52 | 5.95 |
| Exemple comparatif 3 | 7 64 | 2.40 |

Ces résultats montrent que les composés de l'invention présentent de meilleures activités bactériostatiques vis à vis de la bactérie *Staphylococcus aureus* que les composés de l'art antérieur.

### Résultats CMB (µM) :

| **Tableau IV** | **Résultats CMB (**µ**M)** | |
|---|---|---|
| | **Moyenne** | **Ecart-type** |
| Gentamycine | 0.26 | 0 05 |
| Composé 1 | 12.50 | 0.00 |
| Composé 5 | 13.18 | 7.30 |
| Composé 2 | 16.65 | 833 |
| Exemple comparatif 1 | 26.33 | 13 39 |
| Exemple comparatif 2 | 16.63 | 4.15 |
| Exemple comparatif 3 | 15.97 | 7.89 |

Ces résultats montrent que les composés de l'invention présentent des activités bactéricides vis à vis de la bactérie *Staphylococcus aureus* équivalentes à celles des composés de l'art antérieur.

Ainsi, ces résultats montrent que les composés de l'invention sont autant bactéricides vis à vis de la bactérie *Staphylococcus aureus* mais sont bien plus bactériostatiques que les composés de l'art antérieur, ce qui globalement signifie que les composés de l'invention présentent de meilleures activités antibactériennes vis à vis de la bactérie *Staphylococcus aureus* que les composés de l'art antérieur.

### EXEMPLE 3

Dans cet exemple, on a illustré diverses formulations concrètes à base des composés selon l'invention.

### A- VOIE ORALE

(a) Comprimé de 0,2 g
   - Composé 1 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé 5 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé 2 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé 4 0,200 g
   - Glycérine 1,000 g
   - Sorbitol à 70% 1,000 g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé 1 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé 2 0,300 g
   - Vaseline blanche codex qsp 100 g
(c) Crème Eau-dans-Huile non ionique
   - Composé 1 0,100 g
   - Mélange d'alcools de lanoline émulsifs, de cires
      et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé 3 0,100 g
   - Polyéthylène glycol (PEG 400) 69,900 g
   - Ethanol à 95% 30,000 g
(e) Onguent hydrophobe
   - Composé 5 0,300 g
   - Miristate d'isopropyle 36,400 g
   - Huile de silicone ("Rhodorsil 47 V 300" vendu
      par RHONE-POULENC) 36,400 g
   - Cire d'abeille 13,600 g
   - Huile de silicone ("Abil 300.000 cst" vendu
      par GOLDSCHMIDT) qsp 100 g
(f) Crème Huile-dans-Eau non ionique
   - Composé 2 1,000 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Utilisation d'une quantité efficace d'au moins un composé de type rétinoïde pour la préparation d'une composition destinée à traiter de manière préventive ou curative les colonisations bactériennes de *Staphylococcus aureus,* ledit composé rétinoïde étant choisi parmi :
acide 6-[3-(1-adamantyl)-4-methoxy-5-hydroxyphenyl]-2-naphtoïque
acide 4-[4-(6-Methoxymethoxy-4'-methyl-biphenyl-2-yl)-but-3-en-1-ynyl]-benzoïque
acide 4-[4-(6-Methoxy4'-methyl-biphenyl-2-yl)-but-3-en-1-ynyl]-benzoïque
acide 6-[2-methyl-4-hydroxy-5-(1-adamantyl)phenyl]-2-naphtoïque
acide 4-[3-(3,5-Di-tert-butyl-4-oxo-cyclohexa-2,5-dienylidene)-prop-1 -ynyl]-benzoïque
acide 2-Hydroxy-4-[4-(4'-methyl-biphenyl-2-yl)but-3-en-1-ynyl]-benzoïque
acide 2-[3-(1-adamantyl)-4-methoxyphenyl)ethynyl]-4-thiophene carboxylique
acide 4-[4-(4,4'-Dimethyl-biphenyl-2-yl)-but-3-en-1-ynyl]-benzoïque
acide 4-Hydroxy-2'-(5,5,8,8-tetramethyl-5,6,7,8-tetrahydro-naphtalen-2-yl)-[1,1';4',1"]terphenyl-4"- carboxylique
acide 2"-Hydroxy-2'-(5,5,8,8-tetramethyl-5,6,7,8-terahydro-naphtalen-2-yl)-[1,1';4',1"] terphenyl-4"-carboxylique
acide 6-(3-Adamantan-1-yl-5-bromo-4-hydroxy-phenyl)-naphtalene-2-carboxylique
acide 4-[4-(6,4'-Dimethyl-biphenyl-2-yl)-but-3-en-1-ynyl]-benzoïque
acide 2'-(4,4-Dimethyl-thiochroman-6-yl)-[1,1';4',1"]terphenyl-4"-carboxylique
acide 2'-(3-Hydroxy-5,5,8,8-tetramethyl-5,6,7,8-tetrahydro-naphtalen-2-yl)-[1,1';4',1"]terphenyl-4"- carboxylique
acide 4-(5,5,8,8-Tetramethyl-5,6,7,8-tetraydro-naphto[2,3-b]thiophen-2-yl)-benzoïque
acide 4-[(6-hydroxy-7-(1-adamantyl)-2-naphtyl]boezoïque
acide 4-[2-Nonyloxyimino-2-(5,5,8,8-tetramethyl-5,6,7,8-tetrahydro-naphtalen-2-yl)-acetylamino]-benzoïque
acide 4-[3-Adamantan-1-yl-4-(2-methoxy-ethoxymethoxy)-phenylethynyl]-2-hydroxy-benzoïque
acide 4-[[3-(1-adamantyl)-4-[(2-methyl-3-hydroxypropyloxy)phenyl]ethynyl]]benzoïque 2-Hydroxymethyl-5-(5,5,8,8-tetramethyl-5,6,7,8-tetrahydro-anthracen-2-yl)-phenol
acide 4-[4-(4'-Propyl-biphenyl-2-yl)-but-3-en-1-ynyl]-benzoïque
acide 2-Hydroxy-4-(3,5,5,8,8-pentamethyl-5,6,7,8-tetrahydro-naphtalen-2-ylselanylethynyl)- benzoïque
acide 2'-(3-Methoxy-5,5,8,8-tetramethyl-5,6,7,8-teoehydro-naphtalen-2-yl)-[1,1';4',1"] terphenyl-4"- carboxylique
acide 6-[Butoxy-(5,5,8,8-tetramethyl-5,6,7,8-tetrahydro-naphtalen-2-yl)-methyl]-naphtalene-2-carboxylique
acide 4-[3-(1,1-Dimethyl-decyl)-4-methoxy-benzoylamino]-benzoïque
acide 4-(3-Adamantan-1-yl-4-hexyloxy-benzoylamino)-benzoïque 6-[3-(1-adamantyl)-4-hydroxyphenyl]-2-naphtylmethanol
acide 2-Hydroxy-4-(5,5,8,8-tetramethyl-5,6,7,8-tetrahydro-anthracen-2-yl)-benzoïque
acide 2-(3-Adamantan-1-yl-4-hydroxy-phenyl)-benzofuran-5-carboxylique
acide 4-[6-methoxyethoxymethoxy-7-(1-adamantyl)-2-naphtyl]salicylique
acide (E)-[4-(5-Methoxymethoxy-4'-methyl-biphenyl-2-yl)-but-3-en-1-ynyl]-benzoïque
acide 4-[4-(3-Methoxy-4'-methyl-biphenyl-2-yl)-but-3-en-1-ynyl]-benzoîque
acide 4-(3,5,5,8,8-Pentamethyl-5,6,7,8-tetrahydro-naphtalen-2-ylselanylethynyl)-benzoïque
acide 2-Methoxymethoxy-2'-(5,5,8,8-tetramethyl-5,6,7,8-tetrahydxo-naphtalen-2-yl)-[1,1';4',1"]terphenyl-4"-carboxylique
acide 2-Hydroxy-2'-(5,5,8,8-tetramethyl-5,6,7,8-tetrahydro-naphtalen-2-yl)-[1,1';4',1"]terphenyl-4"- carboxylique
acide 4-[(Z)-2-(5,6,7,8-tetrahydro-5,5,8,8-tetramethyl-2-naphtyl)-2-undecenamido]benzoïque
acide 4-[4-(4'-Methyl-biphenyl-2-yl)-but-3-en-1-ynyl]-benzoïque
acide 2'-Propoxy-5'-(5,5,8,8-tetramethyl-5,6,7,8-tetrahydro-naphtalen-2-yl)-biphenyl-4-carboxylique
acide 4-[2-Heptyloxyimino-2-(5,5,8,8-tetramethyl-5,6,7,8-tetrahydro-naphtalen-2-yl)-acetylamino]-benzoïque
amide de l'acide 6-(5,5,8,8-Tetramethyl-5,6,7,8-tetrahydro-naphthalen-2-yloxy)-naphthalene-2-carboxylique
(2-dimethylamino-ethyl)-amide de l'acide 6-(3-Adamantan-1-yl-4-methoxy-phenyl)-naphthalene-2-carboxylique
acide 2-Hydroxy-4-(5,5,8,8-tetramethyl-5,6,7,8-tetrahydro-naphtalen-2-ylselanylethynyl)-benzoique
acide 2'-(3-Methoxymethoxy-5,5,8,8-tetramethyl-5,6,7,8-tetrahydro-naphtalen-2-yl)-[1,1';4',1"] terphenyl-4"-carboxylique
acide 3-Hydroxy-2'-(5,5,8,8-tetramethyl-5,6,7,8-tetrahydro-naphtalen-2-yl)-[1,1';4',1"] terphenyl-4"-carboxylique
acide 3-(5'-Adamantan-1-yl-4'-methoxy 2'-methyl-biphenyl-3-yl)-acrylique

2. Utilisation d'une quantité efficace d'au moins un composé de type rétinoïde choisi parmi la liste de composés de la revendication 1 pour la préparation d'une composition destinée à traiter de manière préventive ou curative les aggravations de pathologies provoquées par une colonisation bactérienne de Staphylococcus aureus.

3. Utilisation d'une quantité efficace d'au moins un composé de type rétinoïde choisi parmi la liste de composés de la revendication 1 pour la préparation d'une composition destinée traiter de manière préventive ou curative les surinfections cutanées induites par une colonisation bactérienne de Staphylococcus aureus.

4. Utilisation selon l'une quelconque des revendications 1 à 3, **caractérisée en ce que** ladite composition est une composition de nettoyage de la peau.

5. Utilisation selon les revendications 1 à 3, **caractérisée en ce que** la surinfection cutanée induite par la colonisation bactérienne est le « syndrome de la peau brûlée ».

6. Utilisation selon les revendications 1 à 3, **caractérisée en ce que** la pathologie liée à une colonisation bactérienne est l'impétigo.

7. Utilisation selon les revendications 1 à 3, **caractérisée en ce que** la pathologie liée à une colonisation bactérienne est la dermatite atopique.

8. Utilisation selon la revendication 7, **caractérisée en ce que** le composé est choisi parmi l'un des composés de la liste ci-dessous :
acide 4-[4-(6-Methoxymethoxy-4'-methyl-biphenyl-2-yl)-but-3-en-1-ynyl]-benzïque
acide 4-[4-(6-Methoxy-4'-methyl-biphenyl-2-yl)-but-3-en-1-ynyl]-benzoïque
acide 4-[6-methoxyethoxymethoxy-7-(1-adamentyl)-2-naphtyl]salicylique
acide (E)-4-[4-(5-Methoxymethoxy-4'-methyl-biphenyl-2-yl)-but-3-en-1-ynyl]-benzoïque
acide 4-[4-(3-Methoxy-4'-methyl-biphenyl-2-yl)-but-3-en-1-ynyl]-benzoïque
acide 2-Methoxymethoxy-2'-(5,5,8,8-tetramethyl-5,6,7,8-tetrahydro-naphtalen -2-yl)-[1,1';4',1"]terphenyl-4"-carboxylique
acide 4-[4-(4'-Methyl-biphenyl-2-yl)-but-3-en-1-ynyl]-benzoïque
acide 4-[4-(4'-Propyl-biphenyl-2-yl)-but-3-en-1-ynyl]-benzoïque.

9. Utilisation selon l'une quelconque des revendications précédentes, **caractérisée en ce que** le composé tel que défini dans la revendication 1 est combiné avec d'autres composés à activité de type rétinoïde, avec les vitamines D ou leurs dérivés, avec des corticostéroïdes, avec des anti-radicaux libres, des α-hydroxy ou α-céto acides ou leurs dérivés ou bien encore avec des bloqueurs de canaux ioniques.

10. Utilisation selon l'une quelconque des revendications précédentes, **caractérisée en ce que** le composé tel que défini dans la revendication 1 est utilisé à une concentration comprise entre 0,001% et 10% en poids par rapport au poids total de la composition.

## Claims

1. Use of an effective amount of at least one compound of retinoid type for the preparation of a composition for preventively or curatively treating bacterial colonisations of *Staphylococcus aureus*, said retinoid compound being chosen from:
6-[3-(1-Adamantyl)-4-methoxy-5-hydroxypenyl]-2-naphthoic acid
4-[4-(6-Methoxymethoxy-4'-methylbiphenyl-2-yl)but-3-en-1-ynyl]henzoic acid
4-[4-(6-Methoxy-4'-methylbiphenyl-2-yl)but-3-en-1-ynyl]benzoic acid
6-[2-Methyl-4 -hydroxy-5-(1-adamantyl)phenyl]-2-naphthoic acid
4-[3-(3,5-Di-tert-butyl-4-oxocyclohexa-2,5-dienylidene)prop-1-ynyl]benzoic acid
2-Hydroxy-4-[4-(4'-methylbiphenyl-2-yl)but-3-en-1-ynyl]benzoic acid
2-[3-(1-Adamantyl)-4-methoxyphenyl)ethynyl]-4-thiophenecarboxylic acid
4-[4-(4,4'-Dimethylbiphenyl-2-yl)but-3-en-1-ynyl]benzoic acid
4-Hydroxy-2'-(5,5,8,8-tetramethyl-5,6,7,8-tetrahydronaphthalen-2-yl)[1,1';4'1"]terphenyl-4"-carboxylic acid
2"-Hydroxy-2'-(5,5,8,8-tetramethyl-5,6,7,8-tetrahydronaphthalen-2-yl)[1,1';4',1"]terphenyl-4"-carboxylic acid
6-(3-Adamantan-1-yl-5-bromo-4-hydroxyphenyl)-naphthalene-2-carboxylic acid
4-[4-(6,4'-Dimethylbiphenyl-2-yl)but-3-en-acid
2'-(4,4-Dimethylthiochroman-6-yl)[1,1';4',1"]terphenyl-4"-carboxylic acid
2'-(3-Hydroxy-5,5,8,8-tetramethyl-5,6,7,8-tetrahydronaphthalen-2-yl)[1,1';4'1"]terphenyl-4"-carboxylic acid
4-(5,5,8,8-Tetramerhyl-5,6,7,8-tetrahydronaphtho-[2,3-b]thiophen-2-yl)benzoic acid
4-[5-Hydroxy-7-(1-adamantyl)-2-naphthyl]benzoic acid
4-[2-Nonyloxyimino-2-(5,5,8,8-tetramethyl-5,6,7,8-tetrahydronaphthalen-2-yl)acetylamino]benzoic acid
4-[3-Adaman-1-yl-4-(2-methoxyethoxymethoxy)-phenylethynyl]-2-hydrpxybenzoic acid
4-[[3-(1-Adamantyl)-4-[(2-methyl-3-hydroxypropyloxy)phenyl]ethynyl]]benzoic acid
2-Hydroxymethyl-5-(5,5,8,8-tetramethyl-1-5,6,7,8-tetrahydroanthracen--2-yl)phenol
4-[4-(4'-propylbiphenyl-2-yl)but-3-en-1-ynyl]benzoic acid
2-Hydroxy-4-(3,5,5,8,8-pentamethyl-5,6,7,8-tetrahydronaphthalen-2-ylselanylethynyl)benzoic acid
2'-(3-Methoxy-5,5,8,8-tetramethyl-5,6,7,8-tetrahydronaphthalen-2-yl)[1,1';4',1"]terphenyl-4"-carboxylic acid
6-[Butoxy-(5,5,8,8-tetramethyl-5,6,7,8-tetrahydronaphthalen-2-yl)methyl]naphthalene-2-carboxylic acid
4-[3-(1,1-Dimethldeyl)-4-methoxybenzoylamino]benzoic acid
4-(3-Adamantan-1-yl-4-hexyloxybenzoylamino)benzoic acid
6-[3-(1-Adamantyl)-4-hydroxyphenyl]-2-napthylmethanol 2-Hydroxy-4-(5,5,8,8-tetramethyl-5,6,7,8-tetrahydroanthracen-2-yl)benzoic acid
2-(3-Adamantan-1-yl-4-hydroxyphenyl)benzofuran-5-carboxyic acid
4-[6-Methoxyethoxymethoxy-7-(1-adamantyl)-2-naphthyl]salicylic acid
(E)-4-[4-(5-Methoxymethoxy-4'-methylbiphenyl-2-yl)but-3-en-1-ynyl]benzoic acid
4-[4-(3-Methoxy-4'-methylbiphenyl-2-yl)but-3-en-1-ynyl]benzoic acid
4-(3,5,5,8,8-Pentamethyl-5,6,7,8-tetrahydronaphthalen-2-ylselanylethynyl)benzoic acid
2-Methoxymethoxy-2'-(5,5,8,8-tetramethyl-5,6,7,8-tetrahydronaphthalen-2-yl)[1,1;4'1"]terphenyl-4"-carboxylic acid
2-Hydroxy-2'-(5,5,8,8-tetramethyl-5,6,7,8-tetrahydronaphthalen-2-yl)[1,1';4'1"]terphenyl-4"-carboxylic acid
4-[(Z)-2-(5,6,7,8-tecrahydro-5,5,8,8-tecramethyl-2-naphthyl)-2-undecenamido]benzoic acid
4-[4-(4'-Methylbipheyl-2-yl)but-3-en-1-ynyl]benzoic acid
2'-Popoxy-5'-(5,5,8,8-tetramethyl-5,6,7,8-tetrahydronaphthalen-2-yl)biphenyl-4-carboxylic acid
4-[2-Heptyloxyimino-2-(5,5,8,8-tetramethyl-5,6,7,8-tetrahydronaphthalen-2-yl)acetylamino]benzoic acid
6-(5,5,8,8-Tetramethyl-5,6,7,8-tetrahydronaphthalen-2-yloxy)naphthalene-2-carboxylic acid amide
(2-Dimethylaminoethyl)amide of 6-(3-adamantan-1-yl-4-methoxyphenyl)naphthalene-2-carboxylic acid
2-Hydroxy-4-(5,5,8,8-tetramethyl-5,6,7,8-tetrahydronaphthalen-2-ylselanylethynyl)benzoic acid
2'-(3-Methoxymethoxy-5,5,8,8-tetramethyl-5,6,7,8-tetrahydronapthalen-2-yl)[1,1';4',1"]terphenyl-4"-carboxylic acid
3-Hydroxy-2'-(5,5,8,8-tetramethyl-5,6,7,8-tetrahydronaphthalen-2-yl)[1,1';4',1"]terphenyl-4"-carboxylic acid
3-(5'-Adamantan-1-yl-4'-methoxy-2'-methylbiphenyl-3-yl)acrylic acid.

2. Use of an effective amount of at least one compound of retinoid type chosen from the list of compounds in Claim 1, for the preparation of a composition for preventively or curatively treating aggravations of pathologies caused by a bacteria colonisation of *Staphylococcus aureus*

3. Use of an effective amount of at least one compound of retinoid type chosen from the list of compounds in Claim 1, for the preparation of a composition, for preventively or curatively treating cutaneous overinfections induced by a bacterial colonization of *staphylococcus aureus.*

4. Use according to any one of Claims 1 to 3, **characterized in that** said composition is a skin cleansing composition.

5. Use according to Claims 1 to 3, **characterized in that** the cutaneous overinfection induced by bacteria colonisation is "scalded skin syndrome".

6. Use according to Claims 1 to 3, **characterized in that** the pathology associated with a bacterial colonization is impetigo.

7. Use according to Claims 1 to 3, **characterized in that** the pathology associated with a bacterial colonization is atopic dermatitis.

8. Use according to Claim 7, **characterized in that** the compound is chosen from one of the compounds in the list below:
4-[4-(6-Methoxymethoxy-4'-methylbiphenyl-2-yl)but-3-en-1-ynyl]benzolc acid
4-[4-(6-Methoxy-4'-methylbiphenyl-2-yl)but-3-en-1-yny]jbenzoic acid
4-[6-Methoxyethoxymethoxy-7-(1-adamantyl)-2-naphthyl]salicylic acid
(E)-4-[4-(5-Methoxymethoxy-4'-methylbiphenyl-2-yl)but-3-en-1-ynyl]benzoic acid
4-[4-(3-Methoxy-4'-methylbiphenyl-2-yl)but-3-en-1-ynyl]benzoic acid
2-Methoxymethoxy-2'-(5.5,8,8-tetramethyl-5,6,7,8,-tetrahydronaphthalen-2-yl)[1,1';4'1"]terphenyl-4"-carboxylic acid
4-[4-(4'-Methylbiphenyl-2-yl)but-3-en-1-ynyl]benzoic acid
4-[4-(4'-Propylbiphenyl-2-yl)but-3-en-1-ynyl]benzoic acid.

9. Use according to any one of the preceding claims, **characterized in that** the compound as defined in Claim 1 is combined with other compounds with activity of retinoid type, with D vitamins or derivatives thereof, with corticosteroids, with free-radical scavengers, α-hydroxy or α-keto acids or derivatives thereof, or alternatively with ion-channel blockers.

10. Use according to any one of the preceding claims, **characterized in that** the compound as defined in Claim 1 is used in a concentration of between 0.001% and 10% by weight relative to the total weight of the composition.

## Patentansprüche

1. Verwendung einer wirksamen Menge mindestens einer Verbindung von Retinoidtyp für die Herstellung einer Zusammensetzung, die für die präventive oder kurative Behandlung bakterieller Besiedelung von *Staphylococcus aureus* vorgesehen ist, wobei das Retinoid ausgewählt ist unter;
6-[3-(1-Adamantyl)-4-methoxy-5-hydroxyphenyl]-2-naphthoesäure
4-[4-(6- Methoymethoxy-4'-methyl biphenyl-2-yl)-but-3-en-1-inyl]-benzoesäure
4-[4-(6-Methoxy-4'-methyl-biphenyl-2-yl)-but-3-en-1-inyl]-benzoesäure
6-[2-Methyl-4-hydroxy-5-(1-adamantyl)phenyl]-2-naphthoesäure
4-[3-(3,5-Di-tert-butyl-4-oxo-cyclohexa-2,5-dienyliden)-prop-1-inyl]-benzoesäure
2-Hydroxy-4-[4-(4'-methyl-biphenyl-2-yl)-but-3-en-1-inyl]-benzoesäure
2-[3-(1-Adamantyl)-4-methoxyphenyl)ethinyl]-4-thiophencarbonsäure
4-[4-(4,4'-Dimethyl-biphenyl-2-yl)-but-3-en-1-inyl]-benzoesäure
4-Hydroxy-2'-(5,5,8,8-tetramethyl-5,6,7,8-tetrahydronaphthalin-2-yl)-[1,1';4,1"]terphenyl"4"-carbonsäure
2" -Hydroxy-2'-(5,5,8,8-tetramethyl-5,6,7,8-tetrahydronaphthalin-2-yl)-[1,1';4',1"]terphenyl-4"-carbonsäure
6-(3-Adamantan-1-yl-5-brom-4-hydroxy-phenyl)-naphthalin-2-carbonsäure
4-[4-(6,4'-Dimethyl-biphenyl-2-yl]-but-3-en-1-inyl]-benzoesäure
2'-(4,4-Dimethyl-thiochroman-6-yl)-[1,1';4',1 "]terphenyl-4"-carbonsäure
2-(3-Hydroxy-5,5,8,8-tetramethyl-5,6,7,8-tetrahydronaphthalin-2-yl)-[1,1';4',1"]terphenyl-4"-carbonsäure
4-(5,5,8,8-Tetramethyl-5,6,7,8-tetrahydro-naphto[2,3-b]-thiophen-2-yl)-benzoesäure
4-[(6-Hydroxy-7-(1-adamantyl)-2-naphtyl]benzoesäure
4-[2-Nonyloxyimino-2-(5,5,8,8-tetramethyl-5,6,7,8-tetrahydronaphthalin-2-yl)-acetylamino]-benzoesäure
4-[3-Adamantan-1-yl-4-(2-methoxy-ethoxymethoxy)-phenylethinyl]-2-hydroxy-benzoesäure
4-[[3-(1-adamantyl)-4-[(2-methyl-3-hydroxypropyloxy)phenyl]-ethinyl]]-benzoesäure
2-Hydroxymethyl-5-(5,5,8,8-tetramethyl-5,6,7,8-tetrahydroanthracen-2-yl)-phenol
4-[4-(4'-Propyl-biphenyl-2-yl)-but-3-en-1-inyl]-benzoesäure
2-Hydroxy-4-(3,5,5,8,8-pentamethyl-5,6,7,8-tetrahydronaphthalin-2ylselanylethinyl)-benzoesäure
2'-(3-Methoxy-5,5,8,8-tetramethyl-5,6,7,8-tetrahydronaphthalin-2-yl)-[1,1';4,1"]terphenyl-4"-carbonsäure
6-[Butoxy-(5,5,8,8-tetramethyl-5,6,7,8-tetrahydro-naphthalin-2-yl)-methyl]naphthalin-2-carbonsäure
4-[3-(1,1-Dimethyl-decyl)-4-methoxy-benzoylamino]-benzoesäure
4-(3-Adamantan-1-yl-4-hexyloxy-benzoylamino)-benzoesäure
6-[3-(1-Adamantyl)-4-hydroxyphenyl]-2-naphtylmethanol
2-Hydroxy-4-(5,5,8,8-tetramethyl-5,6,7,8-tetrahydro-anthracen-2-yl)-benzoesäure
2-(3-Adamantan-1-yl-4-hydroxy-phenyl)-benzofuran-5-carbonsäure
4-[6-Methoxyethoxymethoxy-7-(1-adamantyl)-2-naphtyl]-salicylsäure
(E)-4-[4-(5-Methoxymethoxy-4'-methyl-biphenyl-2-yl)-but-3-en-1-inyl]-benzoesäure 4-[4-(3-Methoxy-4'-methyl-biphenyl-2-yl)-but-3-en-1-inyl]-benzoesäure
4-(3,5,5,8,8-Pentamethyl-5,6,7,8-tetrahydro-naphthalin-2-ylselanylethinyl)benzoesäure
2-Methoxymethoxy-2'-(5,5,8,8-tetramethyl-5,6,7,8-tetrahydronaphthalin-2-yl}-[1,1';4',1"]terphpnyl-4"-carbonsäure
2-Hydroxy-2'-(5,5,8,8-tetramethyl-5,6,7,8-tetrahydronaphthalin-2-yl)-[1,1';4',1"]terphenyl-4"-carbonsäure
4-[(Z)-2-(5,6,7,8-tetrahydro-5,5,8,8-tetramethyl-2-naphtyl)-2-undecenamido]benzoesäure
4-[4-(4'-Methyl-biphenyl-2-yl)-but-3-en-1-inyl]-benzoesäure
2'-Propoxy-5-(5,5,8,8-tetramethyl-5,6,7,8-tetrahydronaphthalin-2-yl)-biphenyl-4-carbonsäure
4-[2-Heptyloxyimino-2-(5,5,8,8-tetramethyl-5,6,7,8-tetrahydronaphthalin-2-yl)-acetylamino]-benzoesäure
6-(5,5,8,8-Tetramethyl-5,6,7,8-tetrahydro-naphthalin-2-yloxy)-naphthalin-2-carbonsäurcamid
6-(3-Adamantan-1-yl-4-methoxy-phenyl)-naphthalin-2-carbonsäure-(2-dimethylamino-ethyl)-amid
2-Hydroxy-4-(5,5,8,8-tetramethyl-5,6,7,8-tetrahydro-naphthalin-2-ylselanylethinyl)-benzoesäure
2'-(3-Methoxymethoxy-5,5,8,8-tetramethyl-5,6,7,8-tetrahydronaphthalin-2-yl)-[1,1';4',1"]terphenyl-4"-carbonsäure
3-Hydroxy-2'-(5,5,8,8-tetramethyl-5,6,7,8-tetrahydronaphthalin-2-yl)-[1,1';4',1"]terphenyl-4"-carbonsäure
3-(5'-Adamantan-1-yl-4'-methoxy-2'-methyl-biphenyl-3-yl)-acrylsäure.

2. Verwendung einer wirksamen Menge mindestens einer Verbindung von Retinoidtyp, die aus der Liste der Verbindungen des Anspruchs 1 ausgewählt ist, für die Herstellung einer Zusammensetzung, die dazu vorgesehen ist, die Verschlimmerung von Erkrankungen, die durch eine bakterielle Besiedelung von *Staphylococcus aureus* hervorgerufen werden, präventiv oder kurativ zu behandeln.

3. Verwendung einer wirksamen Menge mindestens einer Verbindung von Retinoidtyp, die aus der Liste der Verbindungen des Anspruchs 1 ausgewählt ist, für die Herstellung einer Zusammensetzung, die dazu vorgesehen ist, Superinfektionen der Haut, die durch eine bakterielle Besiedelung von *Staphylococcus aureus* hervorgerufen werden, präventiv oder kurativ zu behandeln.

4. Verwendung nach einem der Ansprüche 1 bis 3, **dadurch gekennzeichnet, dass** die Zusammensetzung eine Zusammensetzung für die Reinigung der Haut ist.

5. Verwendung nach den Ansprüchen 1 bis 3, **dadurch gekennzeichnet, dass** es sich bei der Superinfektion der Haut, die durch eine bakterielle Besiedelung hervorgerufen wird, um das "Syndrom der verbrühten Haut" handelt.

6. Verwendung nach den Ansprüchen 1 bis 3, **dadurch gekennzeichnet, dass** es sich bei der Erkrankung, die mit einer bakteriellen Besiedelung zusammenhängt, um Impetigo handelt.

7. Verwendung nach den Ansprüchen 1 bis 3, **dadurch gekennzeichnet, dass** es sich bei der Erkrankung, die mit einer bakteriellen Besiedelung zusammenhängt, um Neurodermitis handelt.

8. Verwendung nach Anspruch 7, **dadurch gekennzeichnet, dass** die Verbindung unter den Verbindungen der nachfolgenden Liste ausgewählt ist:
4-[4-(6-Methoxymethoxy-4'-methyl-biphenyl-2-yl)-but-3-en-1-inyl]-benzoesäure
4-[4-(6-Methoxy-4'-methyl-biphenyl-2-yl)-but-3-en-1-inyl]-benzoesäure
4-[6-Methoxethoxymethoxy-7-(1-adamantyl)-2-naphtyl]-salicylsäure
(E)-4-[4-(5-Methoxymethoxy-4'-methyl-biphenyl-2-yl)-but-3-en-1-inyl]-benzoesäure
4-[4-(3-Methoxy-4'-methyl-biphenyl-2-yl)-but-3-en-1-inyl]-benzoesäure
2-Methoxymethoxy-2'-(5,5,8,8-tetramethyl-5,6,7,8-tetrahydronaphthalin-2-yl)-[1,1';4',1"]terphenyl-4"-carbonsäure
4-[4-(4'-Methyl-biphenyl-2-yl)-but-3-en-1-inyl]-benzoesäure
4-[4-(4'-Propyl-biphenyl-2-yl)-but-3-en-1-inyl]-benzoesäure.

9. Verwendung nach einem der vorhergehenden Ansprüche, **dadurch gekennzeichnet, dass** die Verbindung, wie sie in Anspruch 1 definiert ist, mit weiteren Verbindungen mit einer Aktivität vom Retinoidtyp, mit D-Vitaminen oder deren Derivaten, mit Corticosteroiden, mit Radikalfängern für freie Radikale, α-Hydroxysäuren oder α-Ketosäuren oder deren Derivaten oder auch Ionenkanalblockern kombiniert ist.

10. Verwendung nach einem der vorhergehenden Ansprüche, **dadurch gekennzeichnet, dass** die Verbindung, wie sie in Anspruch 1 definiert ist, in einer Konzentration im Bereich von 0,001 bis 10 Gew.-%, bezogen auf das Gesamtgewicht der Zusammensetzung, verwendet wird.
